# EUROPEAN PATENT SPECIFICATION

(11) **EP 2 322 233 B1**
(45) Date of publication and mention of the grant of the patent: **17.07.2013**
(21) Application number: 11154521.6
(22) Date of filing: 13.06.2008
(51) Int. Cl.: A61K 9/51

(54) **Nanoparticle formulations for treating vascular disease.**
Nanopartikel Formulierungen zur Behandlung von vaskulären Krankheiten.
Formulations de nanoparticules pour le traitement des maladies vasculaires.

(30) Priority: 16.08.2007 US 840119
(43) Date of publication of application: 18.05.2011
(62) Divisional of application: 08780819.2
(73) Proprietor: Abbott Cardiovascular Systems Inc., Santa Clara, CA 95054 (US)
(72) Inventor: Ludwig, Florian N., 6030 Ebikon, Luzern (CH); Hossainy, Syed F.A., Hayward, CA 94544 (US); Murase, Katsuyuki, Cupertino, CA 95014 (US); Zhao, Li, Mountain View, CA 94040 (US); Astafieva, Irina, Palo Alto, CA 94306 (US)
(74) Representative: ZBM Patents

(56) References cited:
- WO-A2-2007/025274
- WO-A2-2007/076295

## Description

### FIELD OF THE INVENTION

The present invention relates to nanoparticle-coated medical devices and nanoparticle-containing formulations used for treating a vascular disease.

### BACKGROUND OF THE INVENTION

The traditional method of administering bioactive agents to treat diseases of the internal organs and vasculature has been by systemic delivery. Systemic delivery involves administering a bioactive agent at a discrete location followed by the agent migrating throughout the patient's body including, of course, to the afflicted organ or area of the vasculature. But to achieve a therapeutic amount of the agent at the afflicted site, an initial dose substantially greater than the therapeutic amount must be administered to account for the dilution the agent undergoes as it travels through the body. Systemic delivery introduces the bioactive agent in two ways: into the digestive tract (enteral administration) or into the vascular system (parenteral administration), either directly, such as injection into a vein or an artery, or indirectly, such as injection into a muscle or into the bone marrow. Absorption, distribution, metabolism, excretion and toxicity, the ADMET factors, strongly influence delivery by each of these routes. For enteric administration, factors such as a compound's solubility, its stability in the acidic environs of the stomach and its ability to permeate the intestinal wall all affect drug absorption and therefore its bioavailability. For parenteral delivery, factors such as enzymatic degradation, lipophilic/hydrophilic partitioning coefficient, half-life in circulation, protein binding, etc. will affect the agent's bioavailability.

At the other end of the spectrum is local delivery, which comprises administering the bioactive agent directly to the afflicted site. With localized delivery, the ADMET factors tend to be less important than with systemic administration because administration is essentially directly to the treatment site. Thus, the initial dose can be at or very close to the therapeutic amount. With time, some of the locally delivered bioactive agent may diffuse over a wider region, but that is not the intent of localized delivery, and the diffused agent's concentration will ordinarily be sub-therapeutic, i.e., too low to have a beneficial effect. Nevertheless, localized delivery of bioactive agents is currently considered a state-of-the-art approach to the treatment of many diseases such as cancer and atherosclerosis.

Localized delivery of bioactive agents may also involve using implantable medical devices, e.g., stents. Stents play an important role in a variety of medical procedures such as, for example, percutaneous transluminal coronary angioplasty (PTCA). Stents act as a mechanical intervention to physically hold open and, if desired, expand a passageway within a subject. Problems with the use of stents, however, include thrombosis and restenosis that may present several months after a particular procedure and create a need for additional angioplasty or a surgical by pass operation.

Localized delivery of bioactive agents also includes the targeted delivery of bioactive agent-containing compositions. This method can consist of administering a composition containing a bioactive agent and a targeting moiety designed to interact specifically with a biochemical entity present at, and preferably exclusive to, the afflicted site in the vasculature, as shown in WO 2007/025274 and WO 2007/076295.

The bioactive agent-containing compositions can include nanoparticles. Nanoparticles, whose maximum linear dimension is no greater than about 400 nm, have the ability to penetrate a vessel wall which provides an effective means to deliver a bioactive agent at a disease site. However, a means to administer nanoparticles without losing a substantial fraction to the systemic circulation or to target nanoparticles to an endothelium is lacking in the art.

The present invention provides nanoparticle-containing formulations with enhanced endothelium targetingand methods of using these for the treatment of vascular disease.

### SUMMARY OF THE INVENTION

The invention relates to a formulation that includes a first population of nanoparticles having a density similar to that of blood and a second population of nanoparticles having a density different from that of blood modified to operatively couple to the surface of the first population of nanoparticles, wherein when the second population of nanoparticles is coupled to the first population of nanoparticles a supra-assembly having a density different from that of blood is formed

and, wherein, the first population of nanoparticles contains one or more bioactive agents encapsulated within, adhered to a surface of or integrated into the structure of the nanoparticles. Suitable bioactive agents are selected from the group including a corticosteroid, everolimus, an everolimus derivative, zotarolimus, a zotarolimus derivative, sirolimus, a sirolimus derivative, paclitaxel, biolimus A9, a bisphosphonate, ApoA1, a mutated ApoA1, ApoA1 milano, an ApoA1 mimetic peptide, an ABC A1 agonist, an anti-inflammatory agent, an anti-proliferative agent, an anti-angiogenic agent, a matrix metalloproteinase inhibitor and a tissue inhibitor of metalloproteinase.

In various aspects, the first population of nanoparticles consists of micelles, worm micelles, polymerosomes, polymer particles, liposomes or hydrogel particles.

In one embodiment, the second population of nanoparticles has a density lower than that of blood. In another embodiment, the second population of nanoparticles has a density higher than that of blood.

In various aspects, the second population of nanoparticles consists of biostable or bioabsorbable polymers. The biostable polymers include polyisobutylene, poly-4 methyl pentene, polypropelyne, polyvinylethylene, polybutylene, polydodecyl methacrylate, amorphouse polyethylene or any combination thereof. The bioabsorble polymers include polybutylene succinate, poly glycerol sebacate, poly d,l lactide or any combination thereof.

In various aspects, the second population of nanoparticles can be made of bioabsorbable glass or bioabsorbable silicate.

Another aspect of the invention relates to a formulation for treating a vascular disease involving providing a formulation according to the invention and administering a therapeutically effective amount of the formulation to a vascular disease locale in a patient in need thereof.

Administering the formulation to the vascular disease locale includes intraarterial delivery which can be by percutaneous transluminal coronary arterial delivery or by using a catheter.

The vascular disease to be treated includes atherosclerosis, restenosis, vulnerable plaque and peripheral arterial disease.

Another aspect of the invention relates to a formulation for treating a vascular disease, involving providing a formulation including a plurality of nanoparticles having a density different from that of blood and further including one or more bioactive agents encapsulated within, adhered to a surface of or integrated into the structure of the nanoparticles and administering a bioactiveally effective amount of the formulation to a vascular disease locale in a patient.

In one embodiment, the population of nanoparticles has a density lower than that of blood. In another embodiment, the population of nanoparticles has a density higher than that of blood.

Suitable bioactive agents are described above.

In various aspects, the nanoparticles consist of biostable or bioabsorbable polymers. Suitable biostable and bioabsorbable polymers are described above.

Administration of the formulation can be accomplished as described above. The vascular diseases to be treated are described above.

### DETAILED DESCRIPTION OF THE INVENTION

In many instances, localized intravascular administration of bioactive agents would comprise a significant improvement in the art. But there are special considerations that must be taken into account in the development of a localized, intravascular drug-delivery system. For example, the system should not promote clotting or thrombogenesis. Moreover, the system should take into account the fact that constant blood flow through the vasculature results in rapid dilution of the bioactive agent. The present invention provides nanoparticle- coated implantable medical devices and nanoparticle formulations that can safely be delivered intravascularly and which can be specifically targeted to a disease site to release bioactive agent over a desired length of time.

Specifically, the present invention relates to an implantable medical device that includes a coating containing a plurality of nanoparticles, wherein the nanoparticles include one or more bioactive agents encapsulated within, adhered to a surface of or integrated into the structure of the nanoparticles and further include one or more contrast enhancing agents encapsulated within, adhered to a surface of or integrated into the structure of the nanoparticles.

As used herein, "implantable medical device" refers to any type of appliance that is totally or partly introduced, surgically or medically, into a patient's body or by medical intervention into a natural orifice. The duration of implantation may be essentially permanent, i.e., intended to remain in place for the lifespan of the patient; until the device biodegrades; or until it is physically removed. Presently preferred implantable medical devices include, without limitation, catheters and stents. Stents can be self-expandable stents or balloon- expandable stents. The underlying structure of the device can be of virtually any design. The device can be made of a metallic material or an alloy such as, but not limited to, cobalt chromium alloy (ELGILOY), stainless steel (316L), high nitrogen stainless steel, e.g., BIODUR 108, cobalt chrome alloy L-605, "MP35N", "MP20N" ELASTINITE (Nitinol), tantalum, nickel-titanium alloy, platinum-iridium alloy, gold, magnesium, or a combination thereof. "MP35N" and "MP20N" are trade names for alloys of cobalt, nickel, chromium and molybdenum available from Standard Press Steel Co., Jenkintown, PA. "MP35N" consists of 35% cobalt, 35% nickel, 20% chromium, and 10% molybdenum. "MP20N" consists of 50% cobalt, 20% nickel, 20% chromium, and 10% molybdenum. Devices made from bioabsorbable or biostable polymers can also be used with the embodiments of the present invention, and are known to those skilled in the art.

As used herein, "nanoparticle" refers to a microscopic particle, composed of one or more polymers, whose size in nanometers (nm) includes a maximum linear dimension of less than 500 nanometers. As used herein, linear dimension refers to the distance between any two points on the surface of a nanoparticle as measured in a straight line.

Nanoparticles of this invention may be composed of a range of materials including, but not limited to, a biostable polymer, a bioabsorbable polymer or a combination thereof. Biostable refers to polymers that are not degraded *in vivo.* The terms bioabsorbable, biodegradable, and bioerodable, as well as absorbed, degraded and eroded are used interchangeably (unless the context shows otherwise) and refer to polymers that are capable of being degraded or absorbed after being delivered to a disease locale in a patient, e.g., when exposed to bodily fluids such as blood, and that can be gradually resorbed, absorbed, and/or eliminated by the body.

Nanoparticles of the present invention can include biodegradable and bioerodable materials that, after delivery, biodegrade or bioerode within 1.0 second to 100 hours, within 10.0 seconds to 10 hours or within 1 minute to 1 hour. Methods of forming nanoparticles with known degradation rates are known to those skilled in the art; see for example U.S. Patent No. 6,451,338 to Gregoriadis et al., U.S. Patent No. 6,168,804 to Samuel et al. and U.S. Patent No. 6,258,378 to Schneider et al..

Suitable nanoparticles include micelles, worm micelles, liposomes, polymersomes, hydrogel particles and polymer particles.

As used herein, "micelle" refers to a supramolecular aggregate of amphipathic molecules in an aqueous solution. Amphiphilic molecules have two distinct components, differing in their affinity for a solute, most particularly water. The part of the molecule that has an affinity for water, a polar solute, is said to be hydrophilic. The part of the molecule that has an affinity for non-polar solutes such as hydrocarbons is said to be hydrophobic. When amphiphilic molecules are placed in an aqueous solution the hydrophilic moiety seeks to interact with the water while the hydrophobic moiety seeks to avoid the water, i.e., they aggregate at the surface of the water. Amphiphilic molecules that have this effect are known as "surfactants." When the Critical Micelle Concentration (CMC) is reached surfactant molecules will self-assemble into spheres with the hydrophilic ends of the molecules facing out, that is, in contact with the water forming the micelle corona and with the hydrophobic "tails" facing toward the center of the of the sphere.

Worm micelles, as the name suggests, are cylindrical in shape rather than spherical. They are prepared by varying the weight fraction of the hydrophilic polymer block to the total block copolymer molecular weight in the hydrophilic polymer-b-hydrophobic polymer structure. Worm micelles have the potential advantage of not only being bio-inert and stable as are spherical polymeric micelles but also of being flexible. Polyethylene oxide has been used extensively to create worm micelles with a number of hydrophobic polymers such as, without limitation, poly(lactic acid), poly(ε-caprolactone), poly(ethylethylene) and polybutadiene. A representative description of worm micelle formation, characterization and drug loading can be found in Kim, Y., et al., Nanotechnology, 2005, 16:S484-S491. The techniques described there as well as any other that is currently known or may become known in the future can be used in the present invention.

Bioactive agents suspended in an aqueous medium can be entrapped and solubilized in the hydrophobic center of micelles and worm micelles, which can result in an increase in the bioavailability as well as improving the stability in biological surroundings, thereby improving the pharmacokinetics and possibly decreasing the toxicity of the bioactive agent. In addition, because of their nanoscale size, generally from about 5 nm to about 100 nm, micelles have been shown to exhibit spontaneous accumulation in pathological areas with leaky vasculature and impaired lymphatic drainage, a phenomenon known as the Enhanced Permeability and Retention or EPR effect.

As used herein, "liposome" refers to a compartment that is completely enclosed by a bilayer typically composed of phospholipids. Liposomes can be prepared according to standard techniques known to those skilled in the art. For example, without limitation, suspending a suitable lipid, e.g., di-acyl phosphatidyl choline, in an aqueous medium followed by sonication of the mixture will result in the formation of liposomes. Alternatively, rapidly mixing a solution of lipid in ethanol-water, for example, by injecting a lipid through a needle into an agitated ethanol-water solution can form lipid vessicles. Liposomes can also be composed of other amphiphilic substances, e.g., shingomyelin or lipids containing poly(ethylene glycol) (PEG).

As used herein, "polymersome" refers to di- or tri-block copolymers that are modified to form bilayer structures similar to liposomes. Depending on the length and composition of the polymers in the block copolymer, polymersomes can be substantially more robust that liposomes. In addition, the ability to control the chemistry of each block of the block copolymer permits tuning of the polymersome's composition to fit the desired application. For example, membrane thickness, i.e., the thickness of the bilayer structure, can be controlled by varying the chain length of the individual blocks. Adjusting the glass transition temperatures of the blocks will affect the fluidity and therefore the permeability of the membrane. Even the mechanism of agent release can be modified by altering the nature of the polymers.

Polymersomes can be prepared by dissolving the copolymer in an organic solvent, applying the solution to a vessel surface and then removing the solvent, which leaves a film of the copolymer on the vessel wall. The film is then hydrated to form polymersomes. Dissolving the block copolymer in a solvent and then adding a weak solvent for one of the blocks, will also create polymersomes. Other means of preparing polymersomes are known to those skilled in the art and are within the scope of this invention.

Polymersomes can be used to encapsulate bioactive agents by including the bioactive agent in the water used to rehydrate the copolymer film. Osmotically driving the bioactive agent into the core of preformed polymersomes, a process known as force loading, may also be employed. Using a double emulsion technique, polymersomes of relative monodispersivity and high loading efficiency are possible. The technique involves using micro fluidic technology to generate double emulsions comprising water droplets surrounded by a layer of organic solvent. These droplet-in-a-drop structures are then dispersed in a continuous water phase. The block copolymer is dissolved in the organic solvent and self-assembles into proto-polymersomes on the concentric interfaces of the double emulsion. Completely evaporating the organic solvent from the shell yields the actual polymersomes. This procedure allows fine control over the polymersome size. In addition, the ability to maintain complete separation of the internal fluids from the external fluid throughout the process allows extremely efficient encapsulation.

As used herein, "hydrogel particle" refers to a usually lightly cross-linked network of polymer chains that is absorbent but stable in an aqueous environment. Hydrogel particles can be used to encapsulate bioactive agents by methods known to those skilled in the art.

As used herein, "polymer particle" refers to a solid or porous particle, in contrast to the shell structure of liposomes and polymersomes and the relatively open structures of hydrogel particles. Methods for adhering a bioactive agent to the surface of or integrating a bioactive agent into the structure of or embedding a bioactive agent into the structure of a polymer particle are known to those skilled in the art.

Polymers that may be used to prepare nanoparticles of this invention include, but are not limited to, poly(N-acetylglucosamine) (Chitin), Chitosan, poly(3-hydroxyvalerate), poly(lactide-co-glycolide), poly(3-hydroxybutyrate), poly(4-hydroxybutyrate), poly(3- hydroxybutyrate-co-3-hydroxyvalerate), polyorthoester, polyanhydride, poly(glycolic acid), poly(glycolide), poly(L-lactic acid), poly(L-lactide), poly(D,L-lactic acid), poly(D,L-lactide), poly(L-lactide-co-D,L-lactide), poly(caprolactone), poly(L-lactide-co-caprolactone), poly(D,L-lactide-co-caprolactone), poly(glycolide-co-caprolactone), poly(trimethylene carbonate), polyester amide, poly(glycolic acid-co-trimethylene carbonate), co-poly(ether- esters) (e.g. PEO/PLA), polyphosphazenes, biomolecules (such as fibrin, fibrin glue, fibrinogen, cellulose, starch, collagen and hyaluronic acid, elastin and hyaluronic acid), polyurethanes, silicones, polyesters, polyolefins, polyisobutylene and ethylene-alphaolefin copolymers, acrylic polymers, vinyl halide polymers and copolymers (such as polyvinyl chloride), polyvinyl ethers (such as polyvinyl methyl ether), polyvinylidene halides (such as polyvinylidene chloride), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates including tyrosine-based polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, carboxymethyl cellulose, fullerenes and lipids.

In certain aspects of the invention, polymers that may be used to prepare nanoparticles of the invention include biostable polymers such as polyisobutylene, poly-4 methyl pentene, polypropelyne, polyvinylethylene, polybutylene, polydodecyl methacrylate, amorphouse polyethylene, parylene, polyvinylidene difluoride or any combination thereof, and bioabsorble polymers such as polybutylene succinate, poly glycerol sebacate, poly d,1 lactide or any combination thereof.

Nanoparticles of the invention can also be made of porous bioabsorbable glass or bioabsorbable silicate.

Nanoparticles of the invention have one or more bioactive agents and one or more contrast enhancing agents encapsulated within, adhered to the surface of or integrated into the structure of the nanoparticles.

As used herein, "encapsulated within" means the bioactive agent or contrast enhancing agent is contained within the outer surface of the nanoparticle.

As used herein, "adhered to the surface of'' means the bioactive agent or contrast enhancing agent is covalently or non-covalently attached to the outer surface of the nanoparticle.

As used herein, "integrated into the structure of'' means the bioactive agent or contrast enhancing agent is part of the chemical structure of the material forming the nanoparticle.

As used herein, "contrast enhancing agent" refers to a chemical agent that can be visualized by an imaging modality and may be used to highlight specific cells and/or areas of the body so that they are more readily observable. Suitable contrast enhancing agents include iodine, barium, barium sulfate and gastrografin.

As used herein, a "bioactive agent" refers to any substance that affects biological processes or that is of medical or veterinary therapeutic or prophylactic utility.

A bioactive bioactive agent refers to a bioactive agent that, when administered in a bioactiveally effective amount to a patient suffering from a disease, has a bioactive beneficial effect on the health and well-being of the patient. A bioactive beneficial effect on the health and well-being of a patient includes, but it not limited to: (1) curing the disease; (2) slowing the progress of the disease; (3) causing the disease to regress; or (4) alleviating one or more symptoms of the disease.

A bioactive agent also refers to an agent that, when administered to a patient, either prevents the occurrence of a disease or disorder or retards the recurrence of the disease or disorder. Such a bioactive agent is often referred to as a prophylactic bioactive agent.

The bioactive agent, also referred to herein as a drug or a therapeutic agent, can be an antiproliferative agent, an anti-inflammatory agent, an antineoplastic, an antimitotic, an antiplatelet, an anticoagulant, an antifibrin, an antithrombin, a cytostatic agent, an antibiotic, an anti-allergic agent, an anti-enzymatic agent, an angiogenic agent, a cyto-protective agent, a cardioprotective agent, a proliferative agent, an ABC A1 agonist or an antioxidant.

Examples of antiproliferative agents include, without limitation, actinomycin D, or derivatives or analogs thereof, i.e., actinomycin D is also known as dactinomycin, actinomycin IV, actinomycin I₁, actinomycin X₁, and actinomycin C₁. Antiproliferative agents can be natural proteineous agents such as a cytotoxin or a synthetic molecule, all taxoids such as taxols, docetaxel, and paclitaxel, paclitaxel derivatives, all olimus drugs such as macrolide antibiotics, rapamycin, everolimus, structural derivatives and functional analogues of rapamycin, structural derivatives and functional analogues of everolimus, FKBP- 12 mediated mTOR inhibitors, biolimus, perfenidone, prodrugs thereof, co-drugs thereof, and combinations thereof. Representative rapamycin derivatives include 40-O-(3- hydroxy)propyl-rapamycin, 40-O-[2-(2-hydroxy)ethoxy]ethyl-rapamycin, or 40-O-tetrazole-rapamycin, 40-epi-(N1-tetrazolyl)-rapamycin, prodrugs thereof, co-drugs thereof, and combinations thereof.

Examples of anti-inflammatory agents include, without limitation, steroidal anti- inflammatory agents, a nonsteroidal anti-inflammatory agent, or a combination thereof. In some embodiments, anti-inflammatory agents include clobetasol, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, deflazacort, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof. The anti-inflammatory agent may also be a biological inhibitor of proinflammatory signaling molecules including antibodies to such biological inflammatory signaling molecules.

Examples of antineoplastics and/or antimitotics include, without limitation, paclitaxel, docetaxel, methotrexate, azathioprine, vincristine, vinblastine, fluorouracil, doxorubicin hydrochloride, and mitomycin.

Examples of antiplatelet, anticoagulant, antifibrin, and antithrombin drugs include, without limitation, sodium heparin, low molecular weight heparins, heparinoids, hirudin, argatroban, forskolin, vapiprost, prostacyclin, prostacyclin dextran, D- phe-pro-arg- chloromethylketone, dipyridamole, glycoprotein IIb/IIIa platelet membrane receptor antagonist antibody, recombinant hirudin and thrombin, thrombin inhibitors such as Angiomax ä (Biogen, Inc., Cambridge, Mass.), calcium channel blockers (such as nifedipine), colchicine, fish oil (omega 3 -fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet- Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetic, 4-amino-2,2,6,6-tetramethylpiperidine-1-oxyl (4- amino-TEMPO), estradiol, anticancer agents, dietary supplements such as various vitamins, and a combination thereof. Examples of such cytostatic substance include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ). An example of an antiallergic agent is permirolast potassium. Other bioactive substances or agents that may be appropriate include alpha-interferon, and genetically engineered epithelial cells.

Examples of cytostatic or antiproliferative agents include, without limitation, angiopeptin, angiotensin converting enzyme inhibitors such as captopril, cilazapril or lisinopril, calcium channel blockers such as nifedipine; colchicine, fibroblast growth factor (FGF) antagonists; fish oil (ω-3-fatty acid); histamine antagonists; lovastatin, monoclonal antibodies such as, without limitation, those specific for Platelet-Derived Growth Factor (PDGF) receptors; nitroprusside, phosphodiesterase inhibitors, prostaglandin inhibitors, suramin, serotonin blockers, steroids, thioprotease inhibitors, triazolopyrimidine (a PDGF antagonist) and nitric oxide.

Examples of antiallergic agents include, without limitation, permirolast potassium.

Examples of other suitable bioactive agents include, without limitation, alpha-interferon, genetically engineered epithelial cells, synthetic inorganic and organic compounds, proteins and peptides, polysaccharides and other sugars, lipids, and DNA and RNA nucleic acid sequences having bioactive, prophylactic or diagnostic activities, nucleic acid sequences include genes, antisense molecules which bind to complementary DNA to inhibit transcription, and ribozymes. Some other examples of suitable bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy; antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta- blockers such as pindolol and antiarrhythmics; antihypertensives; diuretics; vasodilators including general coronary; peripheral and cerebral; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and restenoic reducing agents.

Presently preferred bioactive agents include corticosteroids, everolimus, everolimus derivatives, zotarolimus, zotarolimus derivatives, sirolimus, sirolimus derivatives, paclitaxel, biolimus A9, bisphosphonates, ApoA1, mutated ApoA1, ApoA1 milano, ApoA1 mimetic peptides, ABC A1 agonists, anti-inflammatory agents, anti-proliferative agents, anti-angiogenic agents, matrix metalloproteinase inhibitors and tissue inhibitors of metalloproteinases.

The amount of bioactive agent in a nanoparticle will depend on the required minimum effective concentration (MEC) of the agent and the length of time over which it is desired that the MEC be maintained. For most bioactive agents the MEC will be known to, or readily derivable by, those skilled in the art from the literature. For experimental bioactive agents or those for which the MEC by localized delivery is not known, it can be empirically determined using techniques well-known to those skilled in the art.

The amount of contrast enhancing agent in a nanoparticle will depend on the sensitivity and the specificity of the contrast enhancing agent, the quality of the method of image acquisition as well as the desired signal to noise ratio. The local concentration of nanoparticles at a desired site will also affect the amount of contrast enhancing agent that is to be present in a nanoparticle. Methods of determining a suitable concentration will be discernible by those skilled in the art by using the disclosures herein.

In various aspects of the invention, the contrast enhancing agents enhance one or more imaging modalities such as optical, magnetic resonance, acoustic, ultra-sound, x-ray, gamma-radiation and radioactive-mediated imaging modalities.

Nanoparticles of the invention can have a stealth group operatively coupled to the surface of the nanoparticles, wherein the stealth group increases circulation time of the nanoparticles.

As used herein, "stealth group" refers to a moiety expressed on the surface of a nanoparticle which allows the nanoparticle to evade detection by the immune system, thereby protecting the nanoparticles from being cleared from the host.

Suitable stealth groups include poly(ethylene glycol), an oligosaccharide, a polysaccharide, poly(vinyl pyrrolidone), gluronic acid or polyacrylamide.

As used herein, "operatively coupled" refers to the attachment of a stealth group to the surface of a nanoparticle through either direct or indirect means. For example, it is possible for a stealth group to be directly attached to the surface of a nanoparticle by a portion of the stealth group itself. Alternatively, it is possible that the stealth group is attached to the surface of the nanoparticle via an intermediate component that couples the stealth group with the surface of the nanoparticle. Such intermediate components are often referred to as linkers. Linkers are di-functional molecules that can have one moiety that chemically attaches to a nanoparticle and a second moiety that chemically attaches to a stealth group. Suitable intermediate components will be apparent to those skilled in the art: all such intermediate components are within the scope of this invention.

Stealth groups can be localized to the surface of the nanoparticle by anchoring them to the surface. For example, a stealth group can be covalently bonded to the hydrophilic end of an amphiphilic molecule, such as a phospholipid with a hydrophilic spacer region coupled to its headgroup, or an amphiphilic block co-polymer, such as PEG-PLA. These anchored stealth groups may then be localized to the surface of a nanoparticle by co-incubation of the groups with pre-made nanoparticles, or by including these groups during the nanoparticle formulation process, methods of which are known to those skilled in the art.

Nanoparticles of the invention can further include a first functional group with binding affinity for endothelium operatively coupled to the surface of the nanoparticles.

Functional groups of the invention are operatively coupled to the surface of nanoparticles of the invention, as described above with respect to stealth groups.

The first functional group with binding affinity for endothelium includes one or more first peptides, first proteins, first oligonucleotides or any combination thereof.

When the first functional group is a peptide, it can be a peptide with an RGD sequence or an antibody fragment, e.g., without limitation, a Fab fragment, with binding affinity for endothelium. When the first functional group is an oligonucleotide, it can be an aptamer.

When the first functional group is a first protein, it can be an affibody or an antibody.

As used herein, an "affibody" refers to a relatively small synthetic protein molecule that has high binding affinity for a target protein. Affibodies are composed of a three-helix bundle domain derived from the IgG-binding domain of staphylococcal protein A. The protein domain consists of a 58 amino acid sequence, with 13 randomized amino acids affording a range of affibody variants. Despite being significantly smaller than an antibody (an affibody weighs about 6 kDa while an antibody commonly weighs about 150 kDa), an affibody molecule works like an antibody since it's binding site is approximately equivalent in surface area to the binding site of an antibody.

When the first protein is an antibody, it is an anti-intercellular adhesion molecule, an anti-vascular cellular adhesion molecule, an anti-integrin, an anti-platelet endothelial cell adhesion molecule, an anti-thrombomodulin, an anti-e-selectin, an anti-fibronectin, an anti-sialyl-Lewis[b] glycan, an anti-endothelial glycocalyx protein, an anti-cadherin or any combination thereof.

When the first functional group is an oligonucleotide, it can be an aptamer. As used herein, an "aptamer" refers to an oligonucleic acid that has binding affinity for a specific target, e.g., without limitation, a protein, a nucleic acid, a specific whole cell or a particular tissue. Aptamers can be obtained by *in vitro* selection from a large random sequence pool of nucleic acids, although natural aptamers are also encompassed by the present invention. Other methods of producing aptamers are known to those skilled in the art and are within the scope of this invention.

Nanoparticles of the invention can further include a second functional group with binding affinity for surface-expressed molecules on dysfunctional endothelium operatively coupled to the surface of the nanoparticles.

In one aspect, the second functional group is an aptamer. In one embodiment, the aptamer is an anti-junction adhesion molecule or an anti-leukocyte adhesion molecule.

Nanoparticles of the invention can further include a third functional group with binding affinity for vascular cell wall components operatively coupled to the surface of the nanoparticles. The third functional group will allow nanoparticles to bind to vascular cell wall components different from surface-expressed cellular receptors.

The third functional group includes one or more lipids, third peptides, third proteins, third oligonucleotides or any combination thereof. When the third functional group is a lipid, it is an oleic acid, a stearic acid or an oleate derivative. When the third functional group is an oligonucleotide, it can be an aptamer.

When the third functional group is a peptide, it can be an antibody fragment, e.g., without limitation, a Fab fragment, with binding affinity for a vascular cell wall component.

When the third functional group is a protein, it can be an affibody or an antibody. When the protein is an antibody, it is an anti-elastin, an anti-collagen, an anti-tissue factor, an anti-laminin or any combination thereof.

It is to be understood that nanoparticles of the present invention can include one or more contrast enhancing agents encapsulated within, adhered to a surface of or integrated into the structure of the nanoparticles, as described above, but may also optionally include a stealth group, a first functional group a second functional group and/or a third functional group operatively coupled to the surface of the nanoparticle, as described above.

Another aspect of the invention relates to a method for treating a vascular disease involving providing an implantable medical device of the invention and implanting the medical device in a patient. Methods of implanting medical devices are known to those skilled in the art.

Once a coated device is implanted in a patient, the nanoparticles present in the coating will naturally degrade to release bioactive agent at the site of the vascular disease. In certain embodiments, however, nanoparticles of this invention may possess triggered-release capabilities, e.g., they may be heat-, sound- or light-sensitive. Thus, once nanoparticles are localized at a vessel wall, due to their physical presence on the coated device, they can be triggered to release a bioactive agent(s) by heating, light activation, or ultrasound. This may be done locally through a catheter-based intervention by an external device able to produce localized heat within a body, e.g., focused microwave radiation, or globally, e.g., by inducing fever, although in this latter case, the bioactive agent would still be localized by localization of the drug carrier. Methods of forming nanoparticles with triggered release capabilities are known to those skilled in the art.

Nanoparticles of the invention can also be designed, using the appropriate polymer(s), for delayed degradation. In this aspect, as the device coating degrades, the nanoparticles will be released into the vasculature at the site of device implantation. The majority of these nanoparticles will stay localized to the area around the implanted device due to the presence of one or more of the functional groups of the invention, as described above. Specifically, the first functional group with binding affinity for endothelium can secure the nanoparticles to the vessel wall. Similarly, the second functional group with binding affinity for surface- expressed molecules on dysfunctional endothelium can secure the nanoparticles to the vessel wall in the area of a damaged vessel. Furthermore, the third functional group with binding affinity for other vascular cell wall components can also secure the nanoparticles to the vessel wall, in particular to vessel segments which have incomplete endothelium or which are denuded of endothelial cells. In each of the above situations, binding and localization of nanoparticles to a vessel wall will decrease the amount of bioactive agent-containing nanoparticles lost to the systemic circulation.

Once bioactive agent-loaded nanoparticles are localized to the endothelium, and in some cases effectively bound to the endothelium, due to the biodegradation of the nanoparticles bioactive agent will be released, thereby providing a means for treating a vascular disease.

In some situations, as the device coating dissolves, nanoparticles with one or more functional groups can enter the systemic circulation. If these nanoparticles have surface- expressed stealth groups and/or functional groups as described above, they will evade the degradation by the host'-'s immune system and subsequently localize at sites of denuded and/or dysfunctional endothelium, at which point they can be triggered to release bioactive agent.

Another aspect of the invention relates to a formulation that includes a first population of nanoparticles having a density similar to that of blood and a second population of nanoparticles having a density different from that of blood modified to operatively couple to the surface of the first population of nanoparticles, wherein when the second population of nanoparticles is coupled to the first population of nanoparticles a supra-assembly having a density different from that of blood is formed. In one embodiment, the second population of nanoparticles has a density higher than that of blood. In another embodiment, the second population of nanoparticles has a density lower than that of blood.

The disparity in density between blood and the formulation provides a means to localize nanoparticles of the formulation to a vessel wall, as will be described below.

As used herein, "supra-assembly" refers to a molecular assembly that is made up of at least two distinct nanoparticles that are bound to each other, e.g., a nanoparticle having a density similar to that of blood and a nanoparticle having a density lower than that of blood.

As used herein, "density similar to that of blood" refers to a density of approximately 1.0 g/cm³ to 1.2 g/cm³. As used herein, "density higher than that of blood" refers to a density of approximately 1.3 g/cm³ to about 3.0 g/cm³ As used herein, "density lower than that of blood" refers to a density of approximately 0.01 g/cm³ to 0.9 g/cm³.

The first population of nanoparticles includes one or more bioactive agents encapsulated within, adhered to a surface of or integrated into the structure of the nanoparticles, which can be micelles, worm micelles, polymersomes, polymer particles, liposomes or hydrogel particles, as described above. Suitable bioactive agents are also described above.

The second population of nanoparticles can be made of biostable or bioabsorbable polymers. Suitable biostable polymers include polyisobutylene, poly-4 methyl pentene, polypropelyne, polyvinylethylene, polybutylene, polydodecyl methacrylate, amorphouse polyethylene or any combination thereof. Suitable bioabsorbable polymers include polybutylene succinate, poly glycerol sebacate, poly d,1 lactide or any combination thereof. Methods of making nanoparticles that include biostable and/or bioabsorbable polymers are known to those skilled in the art.

The second population of nanoparticles can also be made of bioabsorbable glass or bioabsorbable silicate.

A therapeutically effective amount of the formulation can then be administered to a patient to treat a vascular disease. Routes of administration are described above.

As used herein, "patient" refers to any organism that can benefit from the administration of a bioactive agent. In particular, patient refers to a mammal such as a cat, dog, horse, cow, pig, sheep, rabbit, goat or a human being.

As used herein, "treating" refers to the administration of a therapeutically effective amount of a bioactive agent to a patient known or suspected to be suffering from a vascular disease. Bioactive agents useful with this invention are described above.

As used herein, a "therapeutically effective amount" refers to the amount of bioactive agent that has a beneficial effect, which may be curative or palliative, on the health and well- being of a patient with regard to a vascular disease with which the patient is known or suspected to be afflicted. A therapeutically effective amount may be administered as a single bolus, as intermittent bolus charges, as short, medium or long term sustained release formulations or as any combination of these.

As used herein, "known" to be afflicted with a vascular disease refers first to a condition that is relatively readily observable and or diagnosable. An example, without limitation, of such a disease is atherosclerosis, which is a discrete narrowing of a patient's arteries. Restenosis, on the other hand, while in its latter stages, like atherosclerosis, is relatively readily diagnosable or directly observable, may not be so in its nascent stage. Thus, a patient may be "suspected" of being afflicted or of being susceptible to affliction with restenosis at some time subsequent to a surgical procedure to treat an atherosclerotic lesion. Further, while restenosis tends generally to occur at the same locus as a previous atherosclerotic lesion, it may not be exactly so, so a region of a segment of a vessel somewhat distant from the site of the initial atherosclerosis may in fact be the site of restenosis.

As used herein, a "vascular disease locale" refers to the location within a patient's body where an atherosclerotic lesion(s) is present, where restenosis may develop, the site of vulnerable plaque(s) or the site of a peripheral arterial disease.

An atherosclerotic lesion refers to a deposit of fatty substances, cholesterol, cellular waste products, calcium and/or fibrin on the inner lining or intima of an artery.

Restenosis refers to the re-narrowing or blockage of an artery at or near the site where angioplasty or another surgical or interventional procedure was previously performed to remove a stenosis.

Vulnerable plaque on the other hand is quite different from either atherosclerosis or restenosis and would generally come under the designation "suspected" affliction. This is because vulnerable plaque occurs primarily within the wall of a vessel and does not cause prominent protrusions into the lumen of the vessel. It is often not until it is "too late," i.e., until after a vulnerable plaque has broken and released its components into the vessel, that its presence is even known. Numerous methods have and are being investigated for the early diagnosis of vulnerable plaque but to date none have proven completely successful. Thus, the regional treatment of a segment of a vessel suspected of being afflicted with vulnerable plaque may be the best way to address such lesions.

As used herein, "peripheral arterial disease" refers to a condition similar to coronary artery disease and carotid artery disease in which fatty deposits build up in the inner linings of the artery walls thereby restricting blood circulation, mainly in arteries leading to the kidneys, stomach, arms, legs and feet.

After administration of the formulation, supra-assemblies with density lower than that of blood will preferentially localize near a vessel wall due to density mismatch, where the nanoparticles can then release bioactive agent.

Alternatively, supra-assemblies with density higher than that of blood will preferentially localize at the bottom of a vessel which in certain situations can aid in the treatment of vascular disease.

In another embodiment, a plurality of supra-assemblies with a range of densities will be administered to the patient. Depending on the particular spectrum of densities, the plurality will preferentially localize along a length of vessel distal to the site of administration. If the range of densities includes densities both lower and higher than that of blood, the supra-assemblies will preferentially localize both near the 'bottom' and 'ceiling' of a vessel.

Another aspect of the invention relates to a method for treating a vascular disease. The method involves providing a formulation containing a plurality of nanoparticles having a density different from that of blood and further containing one or more bioactive agents encapsulated within, adhered to a surface of or integrated into the structure of the nanoparticles and administering a therapeutically effective amount of the formulation to a vascular disease locale in a patient.

In one embodiment, the population of nanoparticles has a density higher than that of blood. In another embodiment, the population of nanoparticles has a density lower than that of blood.

Suitable bioactive agents are described above. The nanoparticles are made of biostable or bioabsorbable polymers, as described above. Suitable density ranges of the population of nanoparticles are described above. Suitable methods of administration are known to those skilled in the art.

As described above, upon administration of a formulation containing nanparticles with density lower than that of blood, the nanoparticles will preferentially localize near a vessel wall due to density mismatch, where the nanoparticles can then release bioactive agent. Alternatively, upon administration of a formulation containing nanoparticles with a density higher than that of blood, the nanoparticles will preferentially localize at the bottom of a vessel which in certain situations can aid in the treatment of vascular disease.

## Claims

1. A formulation comprising:
a first population of nanoparticles having a density similar to that of blood; and
a second population of nanoparticles having a density different from that of blood modified to operatively couple to the surface of the first population of nanoparticles, wherein when the second population of nanoparticles is coupled to the first population of nanoparticles a supra-assembly having a density different from that of blood is formed, and,
wherein the first population of nanoparticles comprises one or more bioactive agents encapsulated within, adhered to a surface of or integrated into the structure of the first population of nanoparticles.

2. The formulation according to claim 1, wherein the bioactive agent is selected from the group consisting of a corticosteroid, everolimus, an everolimus derivative, zotarolimus, a zotarolimus derivative, sirolimus, a sirolimus derivative, paclitaxel, biolimus A9, a bisphosphonate, ApoA1, a mutated ApoA1, ApoA1 milano, an ApoA1 mimetic peptide, an ABC A1 agonist, an anti-inflammatory agent, an anti- proliferative agent, an anti-angiogenic agent, a matrix metalloproteinase inhibitor and a tissue inhibitor of metalloproteinase.

3. The formulation according to claim 1, wherein the first population of nanoparticles comprise a micelle, a worm micelle, a polymerosome, a polymer particle, a liposome or a hydrogel particle.

4. The formulation according to claim 1, wherein the second population of nanoparticles has a density lower than that of blood.

5. The formulation according to claim 1, wherein the second population of nanoparticles has a density higher than that of blood.

6. The formulation according to claim 1, wherein the second population of nanoparticles comprises a biostable polymer, a bioabsorbable polymer, a bioabsorbable glass or a bioabsorbable silicate.

7. The formulation according to claim 6, wherein the biostable polymer comprises polyisobutylene, poly-4 methyl pentene, polypropelyne, polyvinylethylene, polybutylene, polydodecyl methacrylate, amorphouse polyethylene, parylene, polyvinylidene difluoride or any combination thereof and wherein the bioabsorble polymer comprises polybutylene succinate, poly glycerol sebacate, poly d,l lactide or any combination thereof.

8. The formulation as defined in any one of claims 1-7 for use in treating a vascular disease.

9. The formulation as defined in any one of the claims 1-7 for use in treating a vascular disease, the formulation comprising: a plurality of nanoparticles having a density different from that of blood and further comprising one or more bioactive agents encapsulated within, adhered to a surface of or integrated into the structure of the nanoparticles.

10. The formulation according to claim 9, wherein the bioactive agent is selected from the group consisting of a corticosteroid, everolimus, an everolimus derivative, zotarolimus, a zotarolimus derivative, sirolimus, a sirolimus derivative, paclitaxel, biolimus A9, a bisphosphonate, ApoA1, a mutated ApoA1, ApoA1 milano, an ApoA1 mimetic peptide, an ABC A1 agonist, an anti-inflammatory agent, an anti-proliferative agent, an anti-angiogenic agent, a matrix metalloproteinase inhibitor and a tissue inhibitor of metalloproteinase.

11. The formulation according to claim 9, wherein the plurality of nanoparticles has a density lower than that of blood.

12. The formulation according to claim 9, wherein the plurality of nanoparticles has a density higher than that of blood.

13. The formulation according to claim 9, wherein the nanoparticles comprise a biostable polymer, a bioabsorbable polymer, a bioabsorbable glass, or a bioabsorbable silicate.

14. The formulation according to claim 12, wherein the biostable polymer comprises polyisobutylene, poly-4 methyl pentene, polypropelyne, polyvinylethylene, polybutylene, polydodecyl methacrylate, amorphouse polyethylene or any combination thereof, and wherein the bioabsorble polymer comprises polybutylene succinate, polyglycerol sebacate, poly d,l lactide or any combination thereof.

## Patentansprüche

1. Zubereitung umfassend:
eine erste Nanopartikelgesamtheit mit einer Dichte, die ähnlich ist wie die Blutsdichte; und
eine zweite Nanopartikelgesamtheit mit einer Dichte, die verschieden ist von der Blutsdichte,
die modifiziert ist, um operativ mit der Oberfläche der ersten Nanopartikelgesamtheit verbunden zu werden, wobei, wenn die zweite Nanopartikelgesamtheit mit der ersten Nanopartikelgesamtheit verbunden wird, dann wird ein Supraaufbau gebildet, deren Dichte verschieden ist von der Blutsdichte, und,
wobei die erste Nanopartikelgesamtheit eine oder mehrere bioaktive Wirkstoffe umfasst, die darin verkapselt sind, die auf einer Oberfläche der Nanopartikel angehaftet sind oder die in der von der Nanopartikelgesamtheit gebildeten Struktur integriert sind.

2. Zubereitung nach Anspruch 1, wobei der bioaktive Wirkstoff ausgewählt wird aus der Gruppe bestehend aus: einem Corticosteroid, Everolimus, einem Everolimusderivat, Zotarolimus, einem Zotarolimusderivat, Sirolimus, einem Sirolimusderivat, Paclitaxel, Biolimus A9, einem Bisphosphonat, ApoA1, einem mutierten ApoA1, ApoA1 Milano, einem ApoA1 mimetischen Peptid, einem ABC A1 Agonisten, einem entzündungshemmenden Mittel, einem proliferationshemmenden Mittel, einem antiangiogenen Mittel, einem Inhibitor der Matrix-Metalloproteinase und einem Gewebeinhibitor der Metalloproteinase.

3. Zubereitung nach Anspruch 1, wobei die erste Nanopartikelgesamtheit eine Mizelle, eine Wurmmizelle, ein Polymerosom, ein Polymerpartikel, ein Liposom oder ein Hydrogelpartikel umfasst.

4. Zubereitung nach Anspruch 1, wobei die Dichte der zweiten Nanopartikelgesamtheit geringer ist als die Blutsdichte.

5. Zubereitung nach Anspruch 1, wobei die Dichte der zweiten Nanopartikelgesamtheit höher ist als die Blutsdichte.

6. Zubereitung nach Anspruch 1, wobei die zweite Nanopartikelgesamtheit ein biostabiles Polymer, ein bioresorbierbares Polymer, ein bioresorbierbares Glas oder ein bioresorbierbares Silicat umfasst.

7. Zubereitung nach Anspruch 6, wobei das biostabile Polymer Polyisobutylen, Poly-4-methylpenten, Polypropylen, Polyvinylethylen, Polybutylen, Polydodecylmethacrylat, amorphes Polyethylen, Parylen, Polyvinylidendifluorid oder eine beliebige Kombination davon enthält und wobei das bioresorbierbare Polymer Polybutylensuccinat, Polyglycerolsebacat, Poly-(D,L-lactid) oder eine beliebige Kombination davon enthält.

8. Zubereitung nach einem der Ansprüche 1 bis 7 zur Verwendung bei der Behandlung einer Gefäßerkrankung.

9. Zubereitung nach einem der Ansprüche 1 bis 7 zur Verwendung bei der Behandlung einer Gefäßerkrankung, wobei die Zubereitung folgendes umfasst: eine Vielzahl von Nanopartikeln mit einer Dichte, die verschieden ist von der Blutsdichte und ferner umfassend eine oder mehrere bioaktive Wirkstoffe, die darin verkapselt sind, die auf einer Oberfläche der Nanopartikel angehaftet sind oder die in der von den Nanopartikeln gebildeten Struktur integriert sind.

10. Zubereitung nach Anspruch 9, wobei der bioaktive Wirkstoff ausgewählt wird aus der Gruppe bestehend aus: einem Corticosteroid, Everolimus, einem Everolimusderivat, Zotarolimus, einem Zotarolimusderivat, Sirolimus, einem Sirolimusderivat, Paclitaxel, Biolimus A9, einem Biphosphonat, ApoA1, einem mutierten ApoA1, ApoA1 Milano, einem ApoA1 mimetischen Peptid, einem ABC A1 Agonisten, einem entzündungshemmenden Mittel, einem proliferationshemmenden Mittel, einem antiangiogenen Mittel, einem Inhibitor der Matrix-Metalloproteinase und einem Gewebeinhibitor der Metalloproteinase.

11. Zubereitung nach Anspruch 9, wobei die Vielzahl von Nanopartikeln eine Dichte aufweist, die geringer als die Blutsdichte ist.

12. Zubereitung nach Anspruch 9, wobei die Vielzahl von Nanopartikeln eine Dichte aufweist, die höher ist als die Blutsdichte.

13. Zubereitung nach Anspruch 9, wobei die Nanopartikel ein biostabiles Polymer, ein bioresorbierbares Polymer, ein bioresorbierbares Glas, oder ein bioresorbierbares Silicat umfassen.

14. Zubereitung nach Anspruch 12, wobei das biostabile Polymer Polyisobutylen, Poly-4-methylpenten, Polypropylen, Polyvinylethylen, Polybutylen, Polydodecylmethacrylat, amorphes Polyethylen, oder eine beliebige Kombination davon enthält und wobei das bioresorbierbare Polymer Polybutylensuccinat, Polyglycerolsebacat, Poly-(D,L-lactid) oder eine beliebige Kombination davon enthält.

## Revendications

1. Préparation comprenant:
une première population de nanoparticules ayant une densité similaire à celle du sang ;
et une deuxième population de nanoparticules ayant une densité différente à celle du sang
modifiée pour être couplée opérationnellement à la surface de la première population de nanoparticules, dans laquelle lorsque la deuxième population de nanoparticules est couplée à la première population de nanoparticules un supra-assemblage ayant une densité différente de celle du sang est formé, et,
dans laquelle la première population de nanoparticules comprend un ou plusieurs agents bioactifs encapsulés à l'intérieur, adhérés à une surface de ou intégrés dans la structure de la première population de nanoparticules.

2. Préparation selon la revendication 1, dans laquelle l'agent bioactif est choisi dans le groupe constitué d'un corticostéroïde, l'évérolimus, un dérivé de l'évérolimus, le zotarolimus, un dérivé du zotarolimus, le sirolimus, un dérivé du sirolimus, le paclitaxel, le biolimus A9, un bisphosphonate, l'ApoA1, un ApoA1 muté, l'ApoA1 Milano, un peptide mimétique de l'ApoA1, un agoniste de l'ABC A1, un agent anti-inflammatoire, an agent antiprolifératif, an agent anti-angiogénique, un inhibiteur de la métalloprotéinase matricielle et un inhibiteur tissulaire de métalloprotéinase.

3. Préparation selon la revendication 1, dans laquelle la première population de nanoparticules comprend une micelle, une micelle vermiforme, un polymérosome, une particule de polymère, un liposome ou une particule d'hydrogel.

4. Préparation selon la revendication 1, dans laquelle la deuxième population de nanoparticules a une densité inférieure à celle du sang.

5. Préparation selon la revendication 1, dans laquelle la deuxième population de nanoparticules a une densité supérieure à celle du sang.

6. Préparation selon la revendication 1, dans laquelle la deuxième population de nanoparticules comprend un polymère biostable, un polymère bioabsorbable, un verre bioabsorbable ou un silicate bioabsorbable.

7. Préparation selon la revendication 6, dans laquelle le polymère biostable comprend du polyisobutylène, du poly-4-méthylpentène, du polypropylène, du polyvinyléthylène, du polybutylène, du polydodécylméthacrylate, du polyéthylène amorphe, du parylène, du difluorure de polyvinylidène ou une combinaison quelconque de ceux-ci et dans laquelle le polymère bioabsorbable comprend du succinate de polybutylène, du poly(glycérol sébacate), du poly-d,l-lactide ou une combinaison quelconque de ceux-ci.

8. Préparation selon l'une quelconque des revendications 1 à 7 destinée à être utilisée dans le traitement d'une maladie vasculaire.

9. Préparation selon l'une quelconque des revendications 1 à 7 destinée à être utilisée dans le traitement d'une maladie vasculaire, comprenant ladite formulation : une pluralité de nanoparticules ayant une densité différente de celle du sang et comprenant en outre un ou plusieurs agents bioactifs encapsulés à l'intérieur, adhérés à une surface de ou intégrés dans le structure des nanoparticules.

10. Préparation selon la revendication 9, dans laquelle l'agent bioactif est choisi dans le group constitué par un corticostéroïde, l'évérolimus, un dérivé de l'évérolimus, le zotarolimus, un dérivé du zotarolimus, le sirolimus, un dérivé du sirolimus, le paclitaxel, le biolimus A9, un bisphosphonate, l'ApoA1, un ApoA1 muté, l'ApoA1 Milano, un peptide mimétique de l'ApoA1, un agoniste de l'ABC A1, un agent anti-inflammatoire, an agent antiprolifératif, an agent anti-angiogénique, un inhibiteur de la métalloprotéinase matricielle et un inhibiteur tissulaire de métalloprotéinase.

11. Préparation selon la revendication 9, dans laquelle la pluralité de nanoparticules a une densité inférieure à celle du sang.

12. Préparation selon la revendication 9, dans laquelle la pluralité de nanoparticules a une densité supérieure à celle du sang.

13. Préparation selon la revendication 9, dans laquelle les nanoparticules comprennent un polymère biostable, un polymère bioabsorbable, un verre bioabsorbable, ou un silicate bioabsorbable.

14. Préparation selon la revendication 12, dans laquelle le polymère biostable comprend du polyisobutylène, du poly-4-méthylpentène, du polypropylène, du polyvinyléthylène, du polybutylène, du polydodécylméthacrylate, du polyéthylène amorphe ou une combinaison quelconque de ceux-ci, et dans laquelle le polymère bioabsorbable comprend du succinate de polybutylène, du poly(glycérol sébacate), du poly-d,l-lactide ou une combinaison quelconque de ceux-ci.
